# EUROPÄISCHE PATENTSCHRIFT

(11) **EP 4 093 503 B1**
(45) Veröffentlichungstag und Bekanntmachung des Hinweises auf die Patenterteilung: **16.09.2026**
(21) Anmeldenummer: 21702103.9
(22) Anmeldetag: 22.01.2021
(51) Int. Cl.: A61N 5/06, A61B 90/98, F21V 19/00, H05B 41/00

(54) **BESTRAHLUNGSVORRICHTUNG**
IRRADIATION APPARATUS
APPAREIL D'EXPOSITION

(30) Priorität: 22.01.2020 CH 712020
(43) Veröffentlichungstag der Anmeldung: 30.11.2022
(73) Patentinhaber: JK-Holding GmbH, 53578 Windhagen (DE)
(72) Erfinder: GERSTENMEIER, Jürgen, 56567 Neuwied (DE)
(74) Vertreter: IPrime Rentsch Kaelin AG
(86) Internationale Anmeldenummer: PCT/IB2021/050493
(87) Internationale Veröffentlichungsnummer: WO 2021/148997

(56) Entgegenhaltungen:
- WO-A1-2004/010885
- JP-A- 2007 273 382
- JP-A- 2010 160 293
- US-A1- 2009 289 582

## Beschreibung

Die vorliegende Erfindung betrifft eine Vorrichtung zur Bestrahlung vor Lebewesen mit geeigneten Lichtmitteln.

### Technologischer Hintergrund

Vorrichtungen zur Bestrahlung von Lebewesen insbesondere des menschlichen Körpers oder von Teilen des menschlichen Körpers mit optischer Strahlung sind bekannt. Diese werden im medizinischen, kosmetischen und/oder therapeutischen Bereich eingesetzt. Im Bereich der Bestrahlung der Haut werden emittierende Anordnungen eingesetzt, deren Strahlung beispielsweise eine photobiologische Wirkung bei einer bestrahlten Person erzeugt. Die Strahlung trifft hierbei auf die Haut einer Person auf, kann aber je nach konkreter Wellenlänge in tiefere Regionen des Körpers eindringen. Die Wirkung umfasst beispielsweise eine Bräunung der Haut, aber auch weitere physiologische und psychologische Effekte resultieren aus der Bestrahlung. Die Strahlung umfasst das Spektrum der ultravioletten (UV-) Strahlung, der sichtbaren (VIS-) Strahlung und der nahen Infrarot (nIR-) Strahlung. Die UV-Strahlung weist hierbei Wellenlängen im Spektrum zwischen 100 nm und ca. 380 nm auf, die VIS-Strahlung weist hierbei Wellenlängen im Spektrum zwischen ca. 380 nm und ca. 780 nm auf, die nIR- Strahlung weist hierbei Wellenlängen im Spektrum zwischen ca. 780 nm und ca. 1400 nm auf. Die genannten Spektren gehen ineinander über. Je nach Anwendung kann die Bestrahlung auf ein Teilspektrum der genannten Spektren konzentriert sein. Hierzu können medizinisch-kosmetische Strahlung emittierende Anordnungen auch dediziert einzelnen Wellenlängen zugeordnet sein, z.B. der UV-Strahlung, die von Strahlröhren erzeugt wird.

*<Aus der Praxis sind Vorrichtungen zur Einwirkung auf die Haut eines Benutzers bekannt, wie sie beispielsweise in Sonnenstudios eingesetzt werden, bei denen eine zu bestrahlende Person zwecks Bräunung ihrer Haut durch Pigmentierung auf einer eine Liegefläche bzw. Abschlussfläche bildenden Abdeckung liegen kann, wobei unterhalb der Abdeckung eine UV-Strahlung emittierende Anordnung mit in der Regel einer Mehrzahl von Strahlröhren, insbesondere Leuchtstoffröhren, angeordnet ist. Meist weisen solche Bräunungsgeräte auch eine weitere Baueinheit mit weiteren Strahlröhren und einer zweiten Abdeckung auf, die gemeinsam auf die zu bestrahlende Person geschwenkt werden können, sodass die Person umlaufend und gleichmässig gebräunt werden kann. >*

**Aus dem Stand der Technik ist** US2009289582A1 **bekannt, dass ein Lampensystem, bestehend aus einem Lampenkopf, einer Lampenanordnung und einem Datenträger beschreibt. Der Datenträger speichert Betriebs- und Identifikationsdaten, die mithilfe eines Datenlesers ausgelesen und von einem Controller verarbeitet werden, um das Lampensystem zu steuern.**

*<Es versteht sich, dass* es *von zentraler Bedeutung ist, dass stets zugelassene Leuchtmittel verwendet werden, wenn sie dem Zweck der Bestrahlung von Lebewesen insbesondere des menschlichen Körpers oder von Teilen des menschlichen Körpers verwendet werden. Insbesondere ist darauf zu achten, dass in einer Bestrahlungsvorrichtung die Leuchtmittel stets eine konstante Leistung aufweisen, und sie gemäss ihrer Lebensdauer ersetzt werden. Im Solarium Bereich, zum Beispiel, wird mit einer Lebensdauer von 100 bis 1000 Stunden Brenndauer, je nach Fabrikationstyp und Einsatzbereich gerechnet. Es gibt Messgeräte, die zur Ermittlung der Leuchtintensität von Leuchtmitteln eingesetzt werden können. Dies setzt jedoch personelle Ressourcen voraus. >*

Der Einsatz der richtigen Leuchtmittel ist zudem für den Erfolg der Bestrahlung von ausschlaggebender Bedeutung.

Somit besteht ein Bedarf nach einer Vorrichtung der eingangs genannten, insbesondere eine Vorrichtung zur Bestrahlung von Lebewesen mit geeigneten Lichtmitteln, bei der die geeigneten Lichtmittel möglichst einfach und klar identifiziert werden können.

Es ist eine Aufgabe der Erfindung, eine Bestrahlungsvorrichtig zur Beaufschlagung mit Licht anzugeben, bei der nur geeignete Leuchtmittel zum Einsatz kommen, insbesondere womit Schäden an Lebewesen oder an der Bestrahlungsvorrichtig vermieden werden können.

Es ist eine weitere besondere Aufgabe der vorliegenden Erfindung eine solche Vorrichtung bereitzustellen, die mindestens einen Nachteil des Bekannter behebt, insbesondere soll eine einfache(s) und kostengünstige(s) Vorrichtung bereitgestellt werden.

### Darstellung der Erfindung

Mindestens eine dieser Aufgaben wird erfindungsgemäss durch eine Vorrichtung und ein Computer Programm Produkt mit den kennzeichnenden Merkmalen der unabhängigen Ansprüche gelöst.

Gemäss einem Aspekt der Erfindung ist eine Bestrahlungsvorrichtig zur Beaufschlagung eines Bestrahlungsobjekts oder eines Lebewesens mit Licht nach Anspruch 1 angegeben. Die Vorrichtung
umfasst mindestens ein Leuchtmittel mit einer Identifikationseinheit, die Leuchtmittel-spezifische Informationen beinhaltet. Weiterhin umfasst die Vorrichtung einen Rahmen, insbesondere einen reflektierenden Rahmen, mit mindestens einer Leuchtmittelfassung, an welchem das mindestens eine Leuchtmittel angeordnet ist. Das Leuchtmittel ist durch formschlüssiges Einfügen in die Leuchtmittelfassung befestigt. Ein definierter oder vorgegebener Bereich ermöglicht ein Detektionsfeld für eine Kommunikationseinrichtung. Die Identifikationseinheit ist an mindestens einem Leuchtmittel derart positionierbar,
dass sich die Identifikationseinheit durch das formschlüssige Einfügen in die Leuchtmittelfassung innerhalb des Detektionsfelds befindet.

In einer besonderen Ausführungsform erstreckt sich das Detektionsfeld in einem Winkel vom Rahmen, insbesondere im Wesentlichen senkrecht vom Rahmen. Dadurch kann eine besonders gute Kommunikation zwischen Identifikationseinheit und Kommunikationseinrichtung erreicht werden. Insbesondere kann eine besonders gute Kommunikation zwischen einem bestimmten Identifikationseinheit und einer bestimmten Kommunikationseinrichtung erreicht werden

Bevorzugt ist der Rahmen metallisch und definiert eine Bestrahlungsrichtung. Der Rahmen kann als Metallwanne ausgebildet sein, so dass eine Abschirmfunktion gegeben ist. In einer besonderen Ausführungsform der vorliegenden Erfindung, kann der Rahmen insbesondere materialbedingt durch abschirmende Eigenschaften die Ausgestaltung des Detektionsfeld beeinflussen.

Im Sinne der vorliegenden Erfindung kann der als Metallwanne ausgebildete Rahmen als reflektierender Rahmen angesehen werden. Alternativ und/oder ergänzend kann der Rahmen mit einer reflektierenden Schicht ausgestaltet sein. Im Kontext der Erfindung ist reflektierend hauptsächlich hinsichtlich der vom Leuchtmittel abgestrahlten elektromagnetischen Strahlung zu verstehen, insbesondere der Reflexion von Licht. Die Reflexionswirkung kann auch mit anderen Mitteln ergänzend oder alternativ zur metallischen Ausgestaltung und/oder Beschichtung erreicht werden. So können Facetten-Reflektoren zum Beispiel vorgesehen sein, deren reflektierende Oberfläche zur verbesserten Lichtreflexion facettenhaft gearbeitet sind, zum Beispiel musterförmig mit Rautenförmigen Flächen. Besonders bevorzugt besteht der Rahmen aus facettiertem, Aluminium-bedampftem Kunststoff.

In einer besonderen Ausführungsform ist der Bereich als Aussparung im Rahmen ausgeführt, bevorzugt auch als Ausfräsung ausgestaltet. Wenn der definierte Bereich nicht-metallisch ist und vorzugsweise aus einem Kunststoff besteht, kann dann eine Verbesserung der induktiven Kopplung zwischen Identifikationseinheit und Kommunikationseinrichtung erreicht werden.

Die Identifikationseinheit enthält eine spezifische Kodierung, sodass jedes Leuchtmittel eindeutig identifiziert werden kann. Sodann ist es möglich über den Betrieb des Leuchtmittels sicher zu entscheiden.

In einer besonderen Ausführungsform umfasst die Bestrahlungsvorrichtig eine Mehrzahl von Leuchtröhren als Leuchtmittel. Diese Leuchtröhren haben eine Längenausdehnung und mindestens ein, in der Regel zwei Endstücke. Im Sinne der vorliegenden Erfindung können als Leuchtröhren handelsübliche Gasentladungsröhren verstanden werden, bei denen zwischen Elektroden durch Anlegen einer Spannung eine Glimmentladung gezündet wird und anschliessend leuchtet. Die Anwendung der erfindungsgemässen Lehre erstreckt sich aber auch LED Leuchtkörper als Leuchtmittel, die zum Beispiel ebenfalls in Röhrenform mit den passenden Steckverbindung z.B. als Ersatz für Gasentladungsröhren verwendet werden können.

In einer besonderen Ausführungsform ist eine Mehrzahl an als Leuchtröhren ausgestaltete Leuchtmittel parallel als Bestrahlungsfeld angeordnet. Jede dieser Leuchtröhren kann über eine individuelle Identifikationseinheit verfügen.

In einer weiteren besonderen Ausführungsform ist die Identifikationseinheit an einer bestimmten Stelle der Längsausdehnung des Leuchtmittels vorgesehen. Besonders bevorzugt bei allen Leuchtmitteln an derselben Stelle. Ebenso zusätzlich oder alternativ besonders bevorzugt im Wesentlichen in der Mitte, insbesondere in der Mitte eines sich längsförmig röhrenförmig erstreckenden Leuchtkörpers des Leuchtmittels.

In einer besonderen Ausführungsform ist die Identifikationseinheit am Aussenumfang eines längsförmig röhrenförmig erstreckenden Leuchtkörpers des Leuchtmittels angebracht. Alternativ ist die Identifikationseinheit am Innenumfang eines längsförmig röhrenförmig erstreckenden Leuchtkörpers des Leuchtmittels angebracht.

In einer besonderen Ausführungsform ist die Identifikationseinheit stoffschlüssig mit dem Leuchtmittel verbunden, insbesondere am Aussenumfang oder am Innenumfang. Dazu kann die Identifikationseinheit zum Beispiel in einer Glaskokille und/oder in einem Kunststoffmantel untergebracht werden und dieser auf den Aussenumfang oder Innenumfang aufgeklebt oder aufgeschweisst werden. Besonders bevorzugt ist die Identifikationseinheit manipulationssicher mit dem Leuchtmittel verbunden. Dies kann zum Beispiel dadurch erreicht werden, dass eine Manipulation der Identifikationseinheit zu einem irreparablen Schaden an diesem führt. So kann die Identifikationseinheit zum Beispiel als RFID-Transponder ausgestaltet sein, dessen Antenne sich flächig auf einem Aussenumfang eines längsförmig röhrenförmig erstreckenden Leuchtkörpers des Leuchtmittels erstreckt, so dass ein Ablöseversuch zu einem Zerbrechen oder Knicken der Antenne führt und somit den Transponder unwirksam macht.

In einer weiteren besonderen Ausführungsform ist die Identifikationseinheit in einer einstückig am Leuchtmittel ausgebildeten Kammer untergebracht.

In einer besonderen Ausführungsform ist die Identifikationseinheit thermostabil untergebracht und/oder thermostabil ausgestaltet. Dies kann zum Beispiel dadurch bewerkstelligt werden, dass das Identifikationseinheit ausgestaltet wird, um in einem erhöhten (relativ zur Zimmertemperatur) Temperaturbereich ohne Einschränkung zu funktionieren. In einer ganz besonderen Ausführungsform ist die Identifikationseinheit thermostabil untergebracht indem sie in einem thermisch im Wesentlichen isolierten Raum im oder am Leuchtmittel untergebracht ist. Dies kann zum Beispiel in der Form einer Glaskokille bewerkstelligt werden, in die Identifikationseinheit gebettet ist, und die über eine Isolationsschicht, zum Beispiel eine Luftschicht verfügt. Alternativ und/oder ergänzend kann die Identifikationseinheit thermostabil ausgestaltet indem sie im Wesentlichen aus Materialien besteht, die ohne sich zu verformen, oder anderweitig in ihren Eigenschaften zu ändern in einem Temperaturbereich von vorzugsweise bis 500 Grad Celsius, vorzugsweise bis 200 Grad Celsius. Im Sinne der vorliegenden Erfindung sei eine Glaskokille als eine einförmig ausgebildetes, hermetisch abgeschlossenes Glasformkörper zu verstehen, der einen inneren Hohlraum umfasst. In diesen Hohlraum würde die Identifikationseinheit untergebracht.

In einer besonderen Ausführungsform ist die Identifikationseinheit als RF-Identifikationseinheit ausgeführt. Besonders bevorzugt ist die RF-Identifikationseinheit so ausgestaltet, dass Informationen lesbar und/oder schreibbar sind. Dies kann mit einem mindestens einmal beschreibbaren Speicher auf der RF-Identifikationseinheit erreicht werden. Insbesondere ist der Speicher wiederholt beschreibbar.

In einer besonderen Ausführungsform umfasst das Leuchtmittel mindestens eine, bevorzugt zwei Endstücke, welche ausgelegt sind mit einem Leuchtmittelsockel der Bestrahlungsvorrichtung in Wirkverbindung zu treten. Bevorzugt weist diese Wirkverbindung eine Steckverbindung auf, bei der mindesten ein Steckfortsatz aus einem Endstück in eine elektrische Verbindung mit einer Buchse des entsprechenden Leuchtmittelsockels gebracht werden kann. In einer besonderen Ausführungsform umfasst das Leuchtmittele eine Mehrzahl an Steckfortsätzen, die neben der Steckverbindung zur elektrischen Konnektivität mit entsprechenden Buchsen des Sockels im montierten Zustand einen Formschluss ermöglichen. In einer besonders bevorzugten Ausführungsform, in der die Leuchtmittel längsförmig röhrenförmig erstreckenden Leuchtkörper umfassen werden die Steckfortsätze durch verdrehen des Leuchtmittels um die Längsachse in einen formschlüssigen Halt überführt. In dieser Ausführungsform können zum Beispiel erste, insbesondere tangential zur Röhrendurchmesser verlaufende, Führungsnuten an den Sokkein vorgesehen sein, welche die Steckfortsätze aufnehmen und anschliessend in zweite, insbesondere radial zum Röhrendurchmesser verlaufende, Führungsnuten überführen. Ein verdrehen des Leuchtmittels um die Längsachse entspräche einem verdrehen entlang der radial zum Röhrendurchmesser verlaufenden Führungsnuten. In tangentialer Richtung wäre so ein Leuchtmittel durch einen Formschluss in seiner Bewegung blockiert.

In einer besonders bevorzugten Ausführungsform ist die Identifikationseinheit dergestalt am Leuchtmittel positioniert, dass sie sich im montierten Zustand durch das formschlüssige Einfügen in die Leuchtmittelfassung innerhalb des Detektionsfelds befindet.

Die Identifikationseinheit ist dergestalt am Leuchtmittel positioniert, dass sie nach einem Verdrehen des Leuchtmittels um die Längsachse um eine Drehung von zwischen 35 und 160 Grad, bevorzugt zwischen 45 und 125 Grad innerhalb des Detektionsfelds zu liegen kommt. In einer besonderen Ausführungsform ist die Identifikationseinheit dergestalt am Leuchtmittel positioniert, dass sie nach einem Ver-drehen des Leuchtmittels orthogonal zur Kommunikationseinrichtung zu liegen kommt. Im Sinne der vorliegenden Erfindung kann eine Verdrehung um die Längsachse zum Beispiel ausgehend von einer Positionierung eines Leuchtmittels mit zwei Pins angesehen werden. Dieses Leuchtmittel kann druch entsprechende Einfuhrfächer oder Nuten zur Aufnahme in eine Lampenfassung vorgesehen sein. In dieser Ausführungsform würde dann Verdrehen um die Längsachse des Leuchtmittels in beide Richtungen, d.h. um zum Beispiel + oder - 45 bis 90 Grad zu einem Verrasten des Leuchtmittels in der Lampenfassung und/oder einer Kontaktierung der Pins mit der Stromzufuhr führen. In diesem konkreten Beispiel wäre dann die Identfikationseinheit hinsichtlich der Ausrichtung der Pins um einen Winkel von zwischen + oder - 45 bis 90 Grad versetzt, so dass die Identfikationseinheit im Verdrehten Zustand im Detektionsfeld zu liegen kommt.

Ein weiterer Vorteil der vorliegenden Erfindung kann darin liegen, dass zum Beispiel bei Leuchtmitteln mit einer definierten Bestrahlungsrichtung ein verkehrtes Montieren unmöglich ist. Ein solches Leuchtmittel könnte zum Beispiel eine reflektieren Oberfläche aufweisen, welche die Bestrahlung nur in eine Richtung ermöglichen, dafür kummulieren. Ein verkehrtes Montieren, d.h. eine Montage bei der die reflektierende Seite in einen Abstrahlbereich weist und somit ein Abstrahlen in diesen Bereich unterbindet wäre nicht möglich, da die Identfikationseinheit sich nicht im Detektionsfeld befände.

In einer besonderen Ausführungsform erstreckt sich das Detektionsfeld in einem Winkel von zwischen 90 und 135 Grad von einer Normalen der Kommunikationseinheit.

In einer besonders bevorzugten Ausführungsform ist die Identifikationseinheit dergestalt am Leuchtmittel positioniert, dass sie nach einem Verdrehen des Leuchtmittels um die Längsachse in einen Abstand von 2 mm oder weniger oder von zwischen ca. 1 und 2 mm zur Kommunikationseinrichtung zu liegen kommt.

Vorteilhaft ist es, wenn der Abstand zwischen der Identifikationseinheit und der Kommunikationseinrichtung grösser ist als der Abstand zwischen der Kommunikationseinrichtung und dem definierten Bereich, da dann die Kommunikationseinrichtung direkt im oder auf dem definierten Bereich platziert werden kann.

In einer weiteren besonderen Ausführungsform ist der Abstand zwischen Identifikationseinheit und der Kommunikationseinrichtung zwischen 1 und 2 mm oder 3 und 9 mm, insbesondere ca. 6 mm und zwischen Kommunikationseinrichtung und dem Rahmen in etwa die Hälfte davon.

Im Kontext der vorliegenden Erfindung ist eine Grössenangabe mit «ca.» als umfassend eine natürliche, herstellungsbedingten Schwankung in der Regel innerhalb von zwischen 0.01 und 5 % zu verstehen.

In einer besonderen Ausführungsform sind mehrere Leuchtmittel mit je einer Identifikationseinheit ausgestattet und jeder Identifikationseinheit eine Kommunikationseinrichtung zugeordnet, so dass eine eindeutige Zuordnung gegeben ist. Alternativ und oder ergänzend können mehrere Leuchtmittel mit je einer Identifikationseinheit ausgestattet sein, und die Kommunikationseinrichtung zur Bulk-Erkennung ausgestaltet sein, so dass nur eine Kommunikationseinrichtung benötigt ist. Insbesondere kann die Kommunikationseinrichtung ausgestaltet sein, um eine Mehrzahl von Identifikationseinheiten hintereinander zu erkennen, indem ein Anmeldeprotokoll geführt wird, und empfangene Seriennummern protokolliert werden. Nach einmaligem, oder wiederholten Durchläufen kann dann zum Beispiel eine Singulation der einzelnen Identifikationseinheiten gegeben sein. Ergänzend und/oder alternativ können Identifikationseinheit(en) und/oder Kommunikationseinrichtung(en) mit Antikollisionsprotokollen ausgestaltet sein. Besonders bevorzugt ist die Kommunikationseinrichtung und/oder Mehrzahl an Kommunikationseinrichtungen ausgestaltet, um anhand des jeweiligen Detektionsfelds und/oder Mehrzahl an Detektionsfeldern eine Lokalisation der Identifikationseinheit(en) vorzunehmen. Dies kann in einem besonderen Beispiel durch ein Protokoll ausgewählt aus der Gruppe bestehend aus: Triangulation, probabilistische Analyse, deterministische Analyse, Lokalisation, z. Beispiel auf der Basis vorgängiger Kalibrierung nach Abstand, Richtung, etc., Frequenzvariation zwischen den verschiedenen Identifikationseinheiten etc.

In einer besonderen Ausführungsform umfasst die Kommunikationseinrichtung einen Nahfeld Sender/Empfänger. Im Besonderen kann dieser ausgelegt sein, um ein im Detektionsbereich ein Antennenfeld zu erzeugen und eine ein im Detektionsbereich befindliche Identifikationseinheit zu aktivieren. In diesem konkreten Beispiel würde die Identifikationseinheit einen passiven Transponder ohne eigene Stromversorgung umfassen.

In einer besonderen Ausführungsform ist/sind die Kommunikationseinrichtung(en) steuerbar ausgestaltet. Die Steuerbarkeit kann so ausgestaltet sein, dass eine Leistung der Kommunikationseinrichtung modulierbar ist. Insbesondere kann eine modulierbare Leistung als Suchprozess ablaufen, in dem die Kommunikationseinrichtung(en) allfällig im Detektionsbereich befindliche Identifikationseinheit(en) suchen und/oder erkennen.

Die Kommunikationseinrichtung kann mit einer Auswerteeinheit verbunden sein, um die von der Identifikationseinheit gespeicherten Informationen zu verarbeiten.

In einer besonderen Ausführungsform umfasst die Kommunikationseinrichtung und/oder die Auswerteeinheit ein Speichermedium. Das Speichermedium kann zum Beispiel als lokaler Datenspeicher zur Speicherung von Daten, oder auch alternativ oder ergänzend als insbesondere drahtlose Verbindung zu einem externen Speicher vorgesehen sein. In einer weiteren besonderen Ausführungsform umfasst das Speichermedium ein Wechselmedium.

In einer weiteren besonderen Ausführungsform ist das Speichermedium zur Hinterlegung eines eindeutig einem Leuchtmittel zuordenbaren Datenpaket ausgestaltet. Besonders bevorzugt umfasst das Datenpaket Daten ausgewählt aus der Gruppe bestehend aus: spezifische Kodierung, zur eindeutigen Identifizierung des betreffenden Leuchtmittels, Art- und Typ des Leuchtmittels, Datum der Inbetriebnahme und/oder ersten Detektion des Leuchtmittels, Betriebsdauer des Leuchtmittels, etc.

In einer weiteren besonderen Ausführungsform ist Auswerteeinheit ausgestaltet auf der Basis, der auf dem Speichermedium hinterlegten und/oder der von der Kommunikationsrichtung erfassten Informationen eine automatische Steuerung der Leuchtmittel vorzunehmen. So kann zum Beispiel einen Leistungsabfall der Leuchtmittel erfasst und verfolgt werden. Weiter kann die Auswerteeinheit ausgestaltet sein, um das Leuchtmittel in Hinblick auf eine optimierten oder gesetzlich erforderlichen Leistungsabgabe, beispielsweise Bestrahlungsintensität zu steuern. So kann es zum Beispiel vorkommen, dass regulatorische Vorgaben bei der Bestrahlung von Lebewesen insbesondere des menschlichen Körpers oder von Teilen des menschlichen Körpers mit optischer Strahlung im medizinischen, kosmetischen und/oder therapeutischen Bereich jeweils unterschiedlich sind. Ebenfalls können sich zum Beispiel die regulatorischen Vorgaben Länderspezifisch unterscheiden. Mit der Lösung der vorliegenden Erfindung ist es möglich Leuchtmittel und einheitliche Bestrahlungsvorrichtungen vorzusehen, die über die Identifikation der Leuchtmittel und die Steuerung der Leuchtmittel dynamisch an ändernde gesetzliche, regulatorische oder physikalische Anforderungen an einem bestimmten Bestrahlungsvorgang bei der Bestrahlung von Lebewesen mit optischer Strahlung im medizinischen, kosmetischen und/oder therapeutischen Bereich anpassbar sind.

Auch unterstützt die vorliegende Erfindung das Einhalten von physiologischen und/oder gesetlichen Höchstdosen an Bestrahlung in dem zum Beispiel eine gesamte tatsächliche Bestrahlungsintensität einer Mehrzahl von Leuchtmitteln von der Auswerteeinheit geprüft und koordiniert wird.

Weiter kann die Auswerteeinheit in einer besonderen Ausführungsform ausgestaltet sein, um ausgehend von auf der Identifikationseinheit eines Leuchtmittels gespeicherten Informationen eine Mehrzahl an Leuchtmittel im Verbund miteinander zu steuern. In einer besonders bevorzugten Ausführungsform koordiniert die Auswerteeinheit eine Mehrzahl von Leuchtmitteln, um eine möglichst konstante gesamte Bestrahlungsintensität einzuhalten, unabhängig von einem allfälligen Leistungsabfall einzelner Leuchtmittel. Dies kann zum Beispiel dadurch geschehen, dass ein Leistungsabfall eines Leuchtmittels mit einer Mehrleistung anderer Leuchtmittel kompensiert wird.

In einer besonderen Ausführungsform ist die Auswerteeinheit ausgestaltet, um aufgrund der Informationen der Identifikationseinheit eine automatische erste Kalibrierung eines Leuchtmittels vorzunehmen. Diese erste Kalibrierung kann zum Beispiel ein Testlauf mit einem Leuchtmittelstart umfassen und/oder ein Bestrahlungsablauf im Verbund mit weiteren Leuchtmitteln. Die automaische erste Kalibrierung kann auch das setzten von maximalen Leistungen umfassen, um, wie oben geschildert zum Beispiel gesetzliche oder physiologische Vorgaben zu erfüllen. So kann die Auswerteeinheit zum Beispiel die Leistung eines neuen Leuchtmittels drosseln, um eine gewünschte Strahlungsintensität zu erhalten.

In einer besonderen Ausführungsform ist die Auswerteeinheit zur Überwachung der einmal mittels Informationen der Identifikationseinheit identifizierten Leuchtmittel ausgestaltet. Die Überwachung kann Parameter wie Leitungsverbrauch, Startspitze, Betriebsdauer, Betriebszeiten etc. umfassen.

In einer besonderen Ausführungsform sind am Leuchtmittel und/oder an der Leuchtmittelfassung physische Sperren vorgesehen, die ein Einfügen und Fixieren eines Leuchtmittels so dass die Identifikationseinheit nicht im Detektionsbereich zu liegen kommt in die Bestrahlungsvorrichtung verunmöglichen. Solche physischen Sperren können Schlüsselsysteme, Nuten, Wülste, Flansche oder weitere vom Fachmann ohne weiteres ertsellbare Sperren sein, die zum Beispiel ein nicht korrektes Einfügen und/oder Verdrehen eines Leuchtmittels in der Fassung verunmöglichen.

Ein weiterer Aspekt der vorliegenden Erfindung betrifft ein Computerprogramm Produkt, wie im Anspruch 15 definiert.

Das Computerprogramm Produkt ist ausgelegt um ihn einer Bestrahlungsvorrichtung eine Reihe von Schritten durchzuführen. Insbesondere ist das Computerprogramm Produkt zum Betreiben einer Bestrahlungsvorrichtung vorgesehen. Das Computerprogramm Produkt ist ausgelegt als einen ersten Schritt ein Aktivieren einer Kommunikationseinrichtung zur Erzeugung eines Detektionsbereichs durchzuführen. Ein zweiter Schritt umfasst das Detektieren von einer oder mehreren Identifikationseinheiten, die Leuchtmittel spezifische Informationen beinhalten. Ein weiterer Schritt umfasst das Auswerten und/oder Speichern der Leuchtmittel-spezifischen Informationen in einer Auswerteeinheit.

In einer besonderen Ausführungsform umfasst das Computerprogramm Produkt weiter den Schritt der Steuerung der Kommunikationseinrichtung und/oder eines Bestrahlungsprogramms und/oder mindestens eines Bestrahlungsparameters auf der Basis der Leuchtmittel-spezifischen Informationen.

Im Kontext der vorliegenden Erfindung kann das Computerprogramm Produkt Teil der Firmware der Bestrahlungsvorrichtung sein. Insbesondere kann das Computerprogramm Produkt im Steuerungsmodul einer solchen integriert sein.

In einer weiteren besonderen Ausführungsform ist das Computerprogramm Produkt ausgelegt, um mindestens eine Startroutine zu vollführen. Im Sinne der vorliegenden Erfindung kann eine Startroutine zum Beispiel darin bestehen, dass ein Sender/Empfänger, insbesondere ein Nahfeld- Sender/Empfänger der Kommunikationseinrichtung dergestalt aktiviert wird, dass eine im Detektionsbereich befindliche Identifikationseinheit aktiviert wird. Durch das Aktivieren können von der Kommunikationseinheit Leuchtmittel-spezifische Informationen aus der Identifikationseinheit gelesen werden, und einer Auswerteeinheit zugeführt werden und/oder gespeichert werden. In einer weiteren besonderen Ausführungsform ist das Computerprogramm Produkt ausgelegt, um die leuchtmittelspezifischen Informationen mit einer vordefinierten Datenbank an leuchtmittelspezifischen Informationen abzugleichen. Dadurch kann zum Beispiel eine Authentizität eines Leuchtmittels sichergestellt werden. Die leuchtmittelspezifischen Informationen können in diesem Fall zum Beispiel einem bestimmten einzigartigen Code umfassen, der mittels einer Datenbank oder einer entsprechenden Entschlüsselung verifiziert werden kann.

Das Computerprogramm Produkt kann in dieser besonderen Ausführungsform die Verwendung, d. h. das Aktivieren des Leuchtmittels in der Bestrahlungsvorrichtung unterbinden bis dieser Schritt des Verifizieren abgeschlossen worden ist. Weiter kann das Computerprogramm Produkt in einer weiteren besonderen Ausführungsform ein Aktivieren des Leuchtmittels verhindern, wenn zum Beispiel die Betriebsparameter des Leuchtmittels nicht regulatorischen Vorgaben entsprechen und/oder die Betriebsparameter des Leuchtmittels auf einen Defekt hinweisen.

Es ist ein besonderer Vorteil der vorliegenden Erfindung, dass in einem solchen Fall das Computerprogramm Produkt in der Lage ist automatisch mit einem entsprechenden Dienstleister in Verbindung zu treten und die defekten, respektive unzulässigen Betriebsparameter zu melden. In einem solchen Fall kann eine entsprechende Wartung und/oder die Organisation eines entsprechenden Ersatzes in die Wege geleitet werden.

In einer weiteren besonderen Ausführungsform ist das Computerprogramm Produkt ausgelegt, um eine erste Kalibrierung der Leuchtmittel vorzunehmen, die im Detektionsbereich identifiziert wurden. So kann in diesem Beispiel das Computerprogramm Produkt neu eingesetzte Leuchtmittel automatisch erkennen und eine entsprechende Kalibrierung vornehmen. In einem solchen Beispiel könnten alle in einer Bestrahlungsvorrichtung vorgesehenen Leuchtmittel, die im Verbund zur Beaufschlagung eines Patienten und/oder Objekts ausgelegt sind in einem bestimmten Betriebszustand gehalten werden. So kann zum Beispiel das Computerprogramm Produkt einzelne Leuchtmittel dämpfen, während andere in ihrer Leistung erhöht werden. Dadurch kann das Computerprogramm Produkt sicherstellen, dass eine insgesamt über den gesamten Verbund an Leuchtmitteln erzeugte gleichbleibende Strahlungsleistung aufrechterhalten ist.

Insbesondere in medizinischen und/ oder kosmetischen Gerätschaften ist die Sicherheit der Anwendung von Bestrahlungsvorrichtungen besonders wichtig. In diesem Fall kann das Computerprogramm Produkt sicherstellen, dass kein unzulässiger Gebrauch der Leuchtmittel möglich ist. Das Computerprogramm Produkt kann zudem die Erkennung, respektive das Ansteuern der Kommunikationseinrichtung insoweit steuern, als dass diese mit dem Betrieb der Bestrahlungsvorrichtung insgesamt koordiniert werden. In dieser besonderen Ausführungsform kann zum Beispiel das Computerprogramm Produkt ausgelegt sein, um das Aktivieren der Kommunikationseinrichtung mit einem Ausschalten der Bestrahlungsvorrichtung zu koordinieren. Dadurch kann verhindert werden, dass die Kommunikationseinrichtung ihre Detektion in einem elektrischen Magnetfeld durchführen muss, das von den Leuchtmitteln unter Umständen stark verzehrt wird.

Ohne an diese Theorie gebunden zu sein scheint es zumindest im Bereich der elektromagnetischen auf Radiofrequenzen basierenden Detektion zu Störungen des elektromagnetischen Feldes zukommen, wenn zum Beispiel Hochleistungsleuchtmittel ein entsprechendes Strahlenfeld erzeugen.

Ein weiterer Vorteil der vorliegenden Erfindung kann darin gesehen werden, dass unabhängig von länderspezifischen Vorgaben Bestrahlungsvorrichtungen bereitgestellt werden können, die sich dynamisch in ihrem Output durch Erkennen der eingesetzten Leuchtmittel ein zulässiges Bestrahlungsprotokoll umsetzen.

Diese Leuchtmittel werden vom Computerprogramm Produkt, respektive einer Auswerteeinheit erkannt und die entsprechenden Betriebsparameter können automatisch abgespielt werden. So können die Betriebsparameter entweder dynamisch, d. h. nach Erkennen der entsprechenden Leuchtmittel über die Identifikationsmittel auf das Gerät aufgespielt werden, oder ein vordefinierte Satz an Betriebsparameter kann bereits in einer erfindungsgemäßen Bestrahlungsvorrichtung vorliegen und ein Abgleich mit den aus den Identifikationsmitteln herausgelesenen leuchtmittelspezifischen Informationen führt zur Ausstattung der korrekten Betriebsparameter. Im Kontext der vorliegenden Erfindung kann das Computerprogramm Produkt lokal auf der Bestrahlungsvorrichtung gespeichert sein oder alternativ oder ergänzend kann das Computerprogramm Produkt von einem zentralen Rechensystem aus auf periphere Bestrahlungsvorrichtungen durchgeführt werden.

Wenn die Auswerteeinheit Informationen der Identifikationseinheit zur Steuerung des Leuchtmittels verwendet, kann ein sicherer und zuverlässiger Betrieb von nur geeigneten Leuchtmitteln in der Bestrahlungsvorrichtig garantiert werden.

### Figurenbeschrieb

Anhand der nachfolgenden Figuren werden Ausführungsbeispiele der Erfindung beschrieben.
Fig. 1 : zeigt schematisch eine Anordnung mit einer erfindungsgemässen Vorrichtung mit einem Leuchtmittel.
Fig. 2: zeigt schematisch eine Anordnung mit einer weiteren erfindungsgemässen Vorrichtung mit mehreren Leuchtmitteln.
Fig. 3: zeigt eine schematische Darstellung einer Anordnung mit Identifikationseinheit relativ zur Kommunikationseinrichtung und der Möglichkeit der Verdrehung um einen Winkel.

### Ausführung der Erfindung

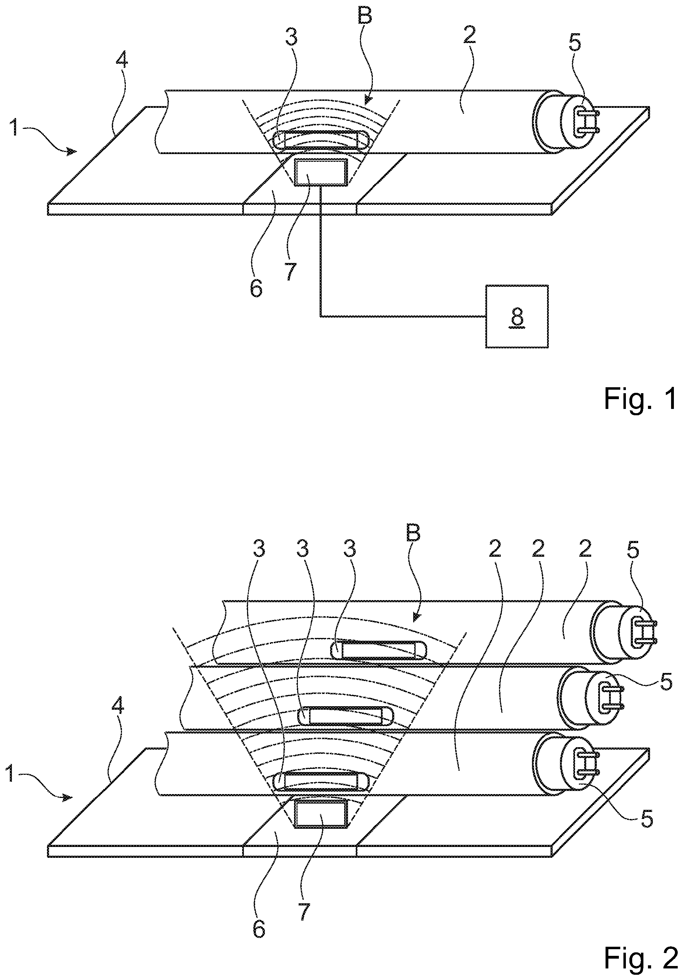

**Fig. 1** zeigt schematisch, wie eine erfindungsgemässe Ausgestaltung einer Bestrahlungsvorrichtig 1 zur Beaufschlagung eines Bestrahlungsobjekts mit Licht ausgeführt ist. Eine solche Bestrahlung dient Menschen oder sonstigen Lebewesens für viele medizinische oder therapeutische Zwecke. Fig. 1 zeigt ein Leuchtmittel 2, beispielsweise eine Lampe, die eine spezielle Strahlung im sichtbaren oder nichtsichtbarem Lichtspektrum aussendet. Auf oder in dem Leuchtmittel 2, die auch als Strahler bezeichnet werden, ist eine Identifikationseinheit 3 angebracht. Das Leuchtmittel 2 ist an einem reflektierenden Rahmen 4 befestigt. Dieser Rahmen 4 ist als Metallwanne 4 ausgeführt, der eine Schirmung vorsieht und die Lichtrichtung zur Bestrahlung vorgibt. Der Rahmen ist vorzugsweise gekrümmt und für eine optimale Bestrahlung ausgelegt. Am Rahmen 4 befindet sich eine Fassung oder Leuchtmittelfassung 5, an welcher das Leuchtmittel 2 befestigt ist. Das Leuchtmittel 2 wird vorzugsweise durch formschlüssiges Einfügen in die Leuchtmittelfassung 5 eingerastet.

Der Rahmen 4 besitzt einen Bereich 6 in dem oder an dem eine Kommunikationseinheit 7 angeordnet ist. An der Kommunikationseinheit 7 ist eine Auswerte- oder Steuereinheit 8 angeschlossen.

Von dem Bereich 6, der als definierter oder vorbestimmter Bereich bezeichnet werden kann, erstreckt sich ein Detektionsfeld B. Das Detektionsfeld B ist in der Regel nicht sichtbar und daher mit gestichelten Linien in der Figur angedeutet. Die Kommunikationseinheit 7 ist in der Lage zum Beispiel durch induktive Kopplung die Identifikationseinheit 3 zu lesen.

Weiterhin ist auch ein Schreiben von Informationen in die Identifikationseinheit 3 durch die Kommunikationseinheit 7 möglich. Bevorzugt wird ein Schrieb/Lesevorgang mit geringer induktiver Kopplung.

Es konnte gezeigt werden, dass die Materiealien einen entscheidenden Einfluss auf das Ergebnis von Lese- und/oder Schreibzyklen haben, die sich im Nahfeld der Kommunikationseinheit 7 befinden. Der Bereich 6 ist vorgesehen, da Störobjekte wie Metallplatten oder Materialien wie Aluminium oder Eisen die induktive Kopplung stören. Die Anordnung einer Kunststoffplatte ermöglicht bereits eine bessere induktive Kopplung, Noch besser ist das Ausfräsen des Rahmens 4 bzw. der Metallwanne und das Einbringen eines nichtleitenden Bereichs 6, der bevorzugt aus Kunststoff besteht.

Ohne an diese Theorie gebunden zu sein, kann es sein, dass das elektromagnetisches Feld in der Nähe von Metallflächen Wirbelströme induziert, die dem anregenden magnetischen Fluss entgegen wirken Dies ist als Lenzsche Regel bekannt und führt zu einer Feldschwächung. Nichtleitende Materialien führen zu keiner wesentlichen Feldschwächung.

Vorteile bringt auch eine Ferritabschirmung, z.B. durch ein dünnes Blättchen zwischen Kommunikationseinheit 7 und dem Metallrahmen 4.

Besonders bevorzugt ist es, wenn die Kommunikationseinheit 7 nahezu parallel zur Identifikationseinheit 3 angeordnet ist, wobei eine geringe Verdrehung zulässig ist. Eine direkte Anordnung übereinander von Identifikationseinheit 3 und Kommunikationseinheit 7

Die Kommunikationseinheit 7 stellt eine Antenne bereit, die durch induktive Kopplung auf die Identifikationseinheit 3, auch als RF-Identifikationseinheit bezeichnet, einwirkt und die in der passiven Identifikationseinheit 3 gespeicherten Informationen auslesen kann. Ein Schreiben von Informationen auf die Identifikationseinheit 3 ist auch möglich.

In HF Bereich können gute Ergebnisse mit der Hochfrequenz-RFID Technologie bei 13,56 MHz und induktiver Kopplung im Nahfeld erzielt werden. Der MHz Bereich ist weltweit verfügbar. Die gösse der Kommunikationseinheit 7 muss auf die Identifikationseinheit 3 abgestimmt sein. Eine sehr große HF-Antenne oder Kommunikationseinheit 7 zum Lesen mehrerer Identifikationseinheiten 3 gleichzeitig lassen wegen metallischer Umgebung keine Vorteile erwarten.

Im UHF Bereich können in Europa die freigegebenen Kanäle K4: 865,7 MHz; K10: 866,9 MHz; K7: 866,3 MHz; oder K14: 867,5 MHz benutzt werden. Je besser die Richteigenschaften der Kommunikationseinheit 7 sind, umso weniger Leistung muss eingespeist werden. Um eine stabile Kommunikation zu ermöglichen, ist die RF Leistung entsprechend anzupassen. Die Sende- und Empfangseigenschaften sind zum Teil Kanal-abhängig, trotz der geringen Frequenzunterschiede.

Fig. 2 zeigt eine schematisch Darstellung einer weiteren Ausführungsform der Bestrahlungsvorrichtung 1 mit mehreren Leuchtmitteln 2. Die Leuchtmittel 2 sind an dem reflektierenden Rahmen 4 befestigt. Jedes Leuchtmittel 2 ist an einer zugehörigen Fassung oder Leuchtmittelfassung 5 befestigt ist. Die Leuchtmittel 2 sind auch hier vorzugsweise durch formschlüssiges Einfügen in die jeweilige Leuchtmittelfassung 5 eingerastet.

Der Rahmen 4 besitzt wiederum den Bereich 6 in dem oder an dem die Kommunikationseinheit 7 angeordnet ist. An der Kommunikationseinheit 7 ist eine Steuereinheit angeschlossen, die jedoch nicht gezeigt ist.

Vom Bereich 6 erstreckt sich das Detektionsfeld B, das hier gleich mehrere Identifikationseinheiten 3 erfasst. Die Kommunikationseinheit 7 ist in der Lage, mehrere Identifikationseinheiten 3 zu lesen oder zu beschreiben. Dies lässt sich bevorzugt mit UHF Einheiten realisieren.

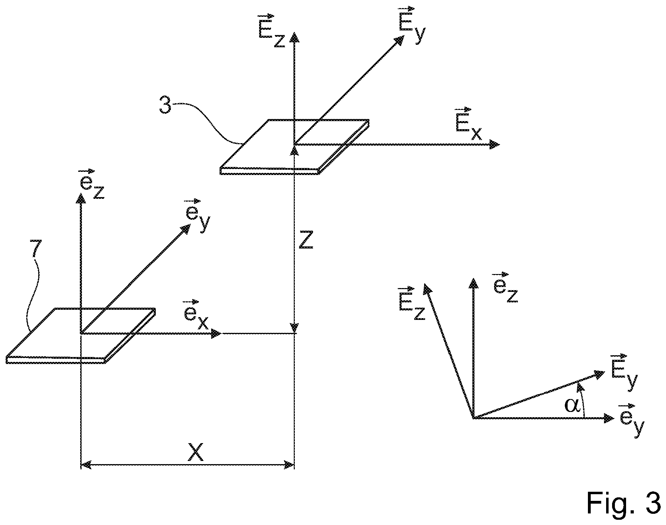

Fig. 3: zeigt eine schematische Darstellung einer Anordnung mit Identifikationseinheit 3 relativ zur Kommunikationseinrichtung 7 und der Möglichkeit der Verdrehung um einen Winkel α. Dieser Winkel α sollte jedoch möglichst gering sein. Dargestellt sind die Vektoren der Identifikationseinheit 3 mit *̅E̅*̅ und der Kommunikationseinrichtung 7 mit *̅e̅*̅. Bei einem minimalen Abstand x, z der Mittelpunkte ergeben sich die besten Resultate für Lese- und/oder Scheibvorgänge.

## Patentansprüche

1. Bestrahlungsvorrichtung (1) zur Beaufschlagung eines Bestrahlungsobjekts mit Licht, umfassend:
mindestens ein Leuchtmittel (2) mit einer Identifikationseinheit (3), die Leuchtmittel-spezifische Informationen beinhaltet;
einen Rahmen (4), insbesondere einen reflektierenden Rahmen (4), mit mindestens einer Leuchtmittelfassung (5), an welchem das mindestens eine Leuchtmittel (2) angeordnet ist durch formschlüssiges Einfügen in die Leuchtmittelfassung (5);
eine Kommunikationseinrichtung (7), und einen Bereich (6), der ein Detektionsfeld (B) der Kommunikationseinrichtung (7) ermöglicht, und **dadurch gekennzeichnet, dass**
die Identifikationseinheit (3) an dem mindestens einem Leuchtmittel (2) derart positioniert ist, so dass sich die Identifikationseinheit (3) durch das formschlüssige Einfügen in die Leuchtmittelfassung (5) innerhalb des Detektionsfelds (B) befindet, wobei die Identifikationseinheit (3) dergestalt am Leuchtmittel (2) positioniert ist, dass sie nach einem Verdrehen des Leuchtmittels (2) in der Lampenfassung (5) um die Längsachse von zwischen 35 und 160 Grad, bevorzugt zwischen 45 und 125 Grad, innerhalb des Detektionsfelds (B) zu liegen kommt.

2. Bestrahlungsvorrichtung gemäss Anspruch 1, wobei das Detektionsfeld (B) sich im Wesentlichen senkrecht vom Rahmen (4) erstreckt.

3. Bestrahlungsvorrichtung gemäss einem der Ansprüche 1 oder 2, wobei der Rahmen (4) metallisch ist und eine Bestrahlungsrichtung definiert.

4. Bestrahlungsvorrichtung gemäss einem der vorgenannten Ansprüche, wobei der Bereich (6) als Aussparung im Rahmen (4) ausgeführt ist, bevorzugt als Ausfräsung.

5. Bestrahlungsvorrichtung gemäss einem der vorgenannten Ansprüche, wobei der Bereich (6) nicht-metallisch ist und vorzugsweise aus einem Kunststoff besteht.

6. Bestrahlungsvorrichtung gemäss einem der vorgenannten Ansprüche, wobei die Identifikationseinheit (3) eine spezifische Kodierung enthält.

7. Bestrahlungsvorrichtung gemäss einem der vorgenannten Ansprüche, wobei die Identifikationseinheit (3) eine RF-Identifikationseinheit ist, von welcher Informationen lesbar sind.

8. Bestrahlungsvorrichtung gemäss einem der vorgenannten Ansprüche, wobei die Identifikationseinheit (3) eine RF-Identifikationseinheit ist, auf welche Informationen schreibbar sind.

9. Bestrahlungsvorrichtung gemäss einem der vorgenannten Ansprüche, wobei der Abstand zwischen der Identifikationseinheit (3) und der Kommunikationseinrichtung (7) grösser ist als der Abstand zwischen der Kommunikationseinrichtung (7) und dem Bereich (6).

10. Bestrahlungsvorrichtung gemäss einem der vorgenannten Ansprüche, wobei der Abstand zwischen der Kommunikationseinrichtung (7) und dem Rahmen (4) zwischen 2 mm und 10 mm beträgt.

11. Bestrahlungsvorrichtung gemäss einem der vorgenannten Ansprüche, wobei mehrere Leuchtmittel (2) mit je einer Identifikationseinheit (3) ausgestattet sind und jeder Identifikationseinheit (3) eine Kommunikationseinrichtung (7) zugeordnet ist.

12. Bestrahlungsvorrichtung gemäss einem der vorgenannten Ansprüche, wobei mehrere Leuchtmittel (2) mit je einer Identifikationseinheit (3) ausgestattet sind und diesen genau eine Kommunikationseinrichtung (7) zugeordnet ist.

13. Bestrahlungsvorrichtung gemäss einem der vorgenannten Ansprüche, wobei die Kommunikationseinrichtung (7) mit einer Auswerteeinheit (8) verbunden ist.

14. Bestrahlungsvorrichtung gemäss Anspruch 13, wobei die Auswerteeinheit (8) Informationen der Identifikationseinheit (3) zur Steuerung des Leuchtmittels (2) verwendet.

15. Computer Programm Produkt zum Betreiben in einer Bestrahlungsvorrichtung zur Beaufschlagung eines Bestrahlungsobjekts mit Licht gemäss Anspruch 1, umfassend Befehle, die bei der Ausführung des Programms durch einen Computer diesen zur Durchführung der folgenden Schritte veranlassen:
a. Aktivieren einer Kommunikationseinrichtung (7) zur Erzeugung eines Detektionsbereichs;
b. Detektieren von einer oder mehrerer Identifikationseinheit(en) (3), die Leuchtmittel-spezifische Informationen beinhalten;
c. Auswerten und/oder speichern der Leuchtmittel-spezifischen Informationen in einer Auswerteeinheit.

16. Computer Programm Produkt gemäss Anspruch 15 weiter umfassend den Schritt:
a. Steuerung der Kommunikationseinrichtung und/oder eines Bestrahlungsprogramms und/oder mindestens eines Bestrahlungsparameters auf der Basis der Leuchtmittel-spezifischen Informationen.

## Claims

1. An irradiation device (1) for irradiating an irradiation object with light, comprising:
at least one lighting means (2) comprising an identification unit (3) which contains lighting means-specific information;
a frame (4), in particular a reflective frame (4), comprising at least one lighting means holder (5), on which the at least one lighting means (2) is arranged by being inserted into the lighting means holder (5) in a form-fitting manner;
a communication apparatus (7), and a region (6) which allows for a detection field (B) of the communication apparatus (7), and **characterized in that**
the identification unit (3) is positioned on at least one lighting means (2) such that the identification unit (3) is positioned within the detection field (B) by being inserted into the lighting means holder (5) in a form-fitting manner, the identification unit (3) being positioned on the lighting means (2) such that, after rotating the lighting means (2) in the lamp holder (5) about the longitudinal axis by between 35 and 160 degrees, preferably between 45 and 125 degrees, it comes to rest within the detection field (B).

2. The irradiation device according to claim 1, wherein the detection field (B) extends substantially perpendicularly from the frame (4).

3. The irradiation device according to any of claims 1 or 2, wherein the frame (4) is metal and defines an irradiation direction.

4. The irradiation device according to any of the preceding claims, wherein the region (6) is designed as a recess in the frame (4), preferably as a milled-out region.

5. The irradiation device according to any of the preceding claims, wherein the region (6) is non-metallic and preferably consists of a plastics material.

6. The irradiation device according to any of the preceding claims, wherein the identification unit (3) contains a specific coding.

7. The irradiation device according to any of the preceding claims, wherein the identification unit (3) is an RF identification unit from which information can be read.

8. The irradiation device according to any of the preceding claims, wherein the identification unit (3) is an RF identification unit to which information can be written.

9. The irradiation device according to any of the preceding claims, wherein the distance between the identification unit (3) and the communication apparatus (7) is greater than the distance between the communication apparatus (7) and the region (6).

10. The irradiation device according to any of the preceding claims, wherein the distance between the communication apparatus (7) and the frame (4) is between 2 mm and 10 mm.

11. The irradiation device according to any of the preceding claims, wherein a plurality of lighting means (2) are each equipped with an identification unit (3) and a communication apparatus (7) is assigned to each identification unit (3).

12. The irradiation device according to any of the preceding claims, wherein a plurality of lighting means (2) are each equipped with an identification unit (3) and exactly one communication apparatus (7) is assigned to each of them.

13. The irradiation device according to any of the preceding claims, wherein the communication apparatus (7) is connected to an evaluation unit (8).

14. The irradiation device according to claim 13, wherein the evaluation unit (8) uses information from the identification unit (3) to control the lighting means (2).

15. A computer program product for operation in an irradiation device for irradiating an irradiation object with light according to claim 1, comprising commands which, when the program is executed by a computer, cause said computer to carry out the following steps:
a. activating a communication apparatus (7) to generate a detection region;
b. detecting one or more identification unit(s) (3) which contain lighting means-specific information;
c. evaluating and/or storing the lighting means-specific information in an evaluation unit.

16. The computer program product according to claim 15, further comprising the step of:
a. controlling the communication apparatus and/or an irradiation program and/or at least one irradiation parameter on the basis of the lighting means-specific information.

## Revendications

1. Dispositif d'illumination (1) destiné à soumettre un objet à un rayonnement lumineux, comprenant :
au moins une source lumineuse (2) dotée d'une unité d'identification (3) contenant des informations spécifiques à la source lumineuse ;
un cadre (4), en particulier un cadre réfléchissant (4), comportant au moins une douille (5) dans laquelle l'au moins une source lumineuse (2) est disposée par insertion avec complémentarité de forme dans la douille (5) ;
un dispositif de communication (7), et une zone (6) permettant un champ de détection (B) du dispositif de communication (7), et **caractérisé en ce que**
l'unité d'identification (3) est positionnée sur l'au moins une source lumineuse (2) de telle sorte que, par son insertion par complémentarité de forme dans la douille (5), l'unité d'identification (3) se trouve à l'intérieur du champ de détection (B), l'unité d'identification (3) étant positionnée sur la source lumineuse (2) de telle sorte qu'après une rotation de la source lumineuse (2) dans la douille (5) autour de son axe longitudinal d'une valeur comprise entre 35 et 160 degrés, de préférence entre 45 et 125 degrés, elle se trouve à l'intérieur du champ de détection (B).

2. Dispositif d'illumination selon la revendication 1, dans lequel le champ de détection (B) s'étend essentiellement perpendiculairement au cadre (4).

3. Dispositif d'illumination selon l'une des revendications 1 ou 2, dans lequel le cadre (4) est métallique et définit une direction d'illumination.

4. Dispositif d'illumination selon l'une des revendications précédentes, dans lequel la zone (6) est réalisée sous la forme d'un évidement dans le cadre (4), de préférence sous la forme d'un fraisage.

5. Dispositif d'illumination selon l'une des revendications précédentes, dans lequel la zone (6) est non métallique et est de préférence constituée d'une matière plastique.

6. Dispositif d'illumination selon l'une des revendications précédentes, dans lequel l'unité d'identification (3) contient un codage spécifique.

7. Dispositif d'illumination selon l'une des revendications précédentes, dans lequel l'unité d'identification (3) est une unité d'identification par radiofréquence (RF) à partir de laquelle des informations peuvent être lues.

8. Dispositif d'illumination selon l'une des revendications précédentes, dans lequel l'unité d'identification (3) est une unité d'identification RF sur laquelle des informations peuvent être écrites.

9. Dispositif d'illumination selon l'une des revendications précédentes, dans lequel la distance entre l'unité d'identification (3) et le dispositif de communication (7) est supérieure à la distance entre le dispositif de communication (7) et la zone (6).

10. Dispositif d'illumination selon l'une des revendications précédentes, dans lequel la distance entre le dispositif de communication (7) et le cadre (4) est comprise entre 2 mm et 10 mm.

11. Dispositif d'illumination selon l'une des revendications précédentes, dans lequel plusieurs sources lumineuses (2) sont équipées chacune d'une unité d'identification (3) et un dispositif de communication (7) est associé à chaque unité d'identification (3).

12. Dispositif d'illumination selon l'une des revendications précédentes, dans lequel plusieurs sources lumineuses (2) sont équipées chacune d'une unité d'identification (3) et un unique dispositif de communication (7) est associé à chacune d'elles.

13. Dispositif d'illumination selon l'une des revendications précédentes, dans lequel le dispositif de communication (7) est relié à une unité d'évaluation (8).

14. Dispositif d'illumination selon la revendication 13, dans lequel l'unité d'évaluation (8) utilise les informations de l'unité d'identification (3) pour commander la source lumineuse (2).

15. Produit de programme informatique destiné à être utilisé dans un dispositif d'illumination pour exposer un objet à la lumière selon la revendication 1, comprenant des instructions qui, lors de l'exécution du programme par un ordinateur, amènent celui-ci à effectuer les étapes suivantes :
a. Activer un dispositif de communication (7) pour générer un champ de détection ;
b. Détecter une ou plusieurs unités d'identification (3) contenant des informations spécifiques à la source lumineuse ;
c. Évaluer et/ou enregistrer les informations spécifiques à la source lumineuse dans une unité d'évaluation.

16. Produit de programme informatique selon la revendication 15, comprenant en outre l'étape suivante :
a. Commande du dispositif de communication et/ou d'un programme d'illumination et/ou d'au moins un paramètre d'illumination sur la base des informations spécifiques à la source lumineuse.
